# EUROPEAN PATENT APPLICATION

(11) **EP 3 381 901 A1**
(43) Date of publication of application: **03.10.2018**
(21) Application number: 16868254.0
(22) Date of filing: 15.09.2016
(51) Int. Cl.: C07D 213/71

(54) **PENTAFLUOROSULFANYLPYRIDYL-GROUP-CONTAINING DIARYLIODONIUM SALT**

(30) Priority: 25.11.2015 JP 2015229881
(71) Applicant: UBE Industries, Ltd., Ube-shi, Yamaguchi 755-8633 (JP)
(72) Inventor: SHIBATA, Norio, Nagoya-shi Aichi 466-0061 (JP); MATSUZAKI, Kohei, Nagoya-shi Aichi 466-0061 (JP); SAITO, Norimichi, Ube-shi Yamaguchi 755-8633 (JP)
(74) Representative: Webster, Jeremy Mark
(86) International application number: PCT/JP2016/077312
(87) International publication number: WO 2017/090305

(57) **Abstract**

Provided is a compound that can easily introduce a 2-pentafluorosulfanylpyridyl group into a target compound. The compound is represented by General Formula (d): 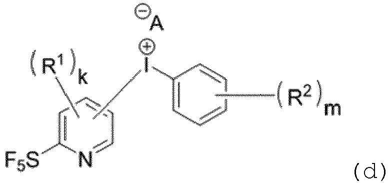 In the formula, k is 1 or 2, R¹ is an alkyl group having 1 or 2 carbon atoms, k is an integer of 0 to 4, R² is a linear or branched alkyl group having 1 to 4 carbon atoms, m is an integer of 0 to 5, and A⁻ is a counter anion.

## Description

### Technical Field

The present invention relates to a pentafluorosulfanylpyridyl group-containing diaryliodonium salt. More specifically, the present invention relates to a pentafluorosulfanylpyridyl group-containing diaryliodonium salt and a method for introducing a pentafluorosulfanyl group-containing pyridyl group into a compound using the pentafluorosulfanylpyridyl group-containing diaryliodonium salt.

### Background Art

A pentafluorosulfanyl group (hereinafter, referred to as an SF₅ group) imparts an electron-withdrawing property and high fat-solubility to a compound. Therefore, an SF₅ group-containing compound is expected to be applied to an electronic material and development of a medical and agricultural chemical. Particularly, synthesis of a compound having an SF₅ group introduced into a pyridine ring frequently used for designing a physiologically active substance is important. It has been reported that an SF₅ group-containing pyridine compound is synthesized by a halogen exchange reaction with silver fluoride following oxidation with a chlorine gas in the presence of potassium fluoride using a disulfide compound as a starting material (Non-Patent Literature 1).

Meanwhile, a diaryliodonium salt is known as a compound that can introduce an aryl group into another compound. The diaryliodonium salt is disclosed in Non-Patent Literatures 2 to 6, for example.

### Citation List

### Non-Patent Literature

Non-Patent Literature 1: Angew. Chem. Int. Ed. 2015, 54, 280.
Non-Patent Literature 2: Chemistry Open 2014, 3, 19
Non-Patent Literature 3: J. Label. Compd. Radiopharm 2007, 50, 452
Non-Patent Literature 4: J. Am. Chem. Soc. 2013, 135, 3772
Non-Patent Literature 5: Eur. J. Org. Chem. 2015, 5919
Non-Patent Literature 6: Chem. Commun. 2015, 51, 2273

### Summary of Invention

### Technical Problem

The inventors have gained an idea that synthesis of a diaryliodonium salt having an SF₅-containing pyridine structure will bring a large advantage in the field of pharmaceuticals and the like. Particularly, a pentafluorosulfanyl group-containing pyridyl group in which an SF₅ group is bonded to a 2-position of a pyridine ring (hereinafter, also referred to as a 2-SF₅Py group) is amphiphilic. Therefore, introduction of the group into a target compound can provide an advantage compound in the field of pharmaceuticals and the like. However, Non-Patent Literatures 1 to 6 do not disclose such a compound. A method described in Non-Patent Literature 1 limits a substrate that can be used, and it is difficult to synthesize such a target compound.

In view of such circumstances, an object of the present invention is to provide a diaryliodonium salt having a 2-SF₅Py group.

### Solution to Problem

The inventors have found that a specific diaryliodonium salt solves the above problems and have completed the present invention. That is, the above problems are solved by the present invention described below.
(1) A diaryliodonium salt having a pentafluorosulfanyl group at a 2-position of a pyridine ring represented by General Formula (d) described later.
(2) The diaryliodonium salt according to (1), in which k is 0, and m is 3 in the formula.
(3) The diaryliodonium salt according to (2), in which R² is a methyl group, an ethyl group, an n-propyl group, an i-propyl group, or a methoxy group.
(4) The diaryliodonium salt according to (1), in which k is 0, and m is 0 in the formula.
(5) A method for manufacturing the diaryliodonium salt according to any one of (1) to (4), including:
   a step of preparing a compound represented by General Formula (b) described later; and
   a step of simultaneously performing an oxidation reaction of the compound and a Friedel-Crafts reaction thereof with a compound represented by General Formula (c) to generate a diaryliodonium salt represented by General Formula (d).
(6) A method for manufacturing a compound represented by General Formula (f), including causing a reaction between a compound represented by General Formula (d) described later and a nucleophilic compound Z to introduce a pyridyl group having a pentafluorosulfanyl group at a 2-position into the nucleophilic compound.
(7) The method for manufacturing the compound according to (6), in which the nucleophilic compound Z is selected from the group consisting of a 1,3-dicarbonyl compound, a phenol compound, an aniline compound, a heterocyclic compound, an alcohol compound, an oximide compound, an aromatic sulfur compound, and an aromatic cyan compound.

### Advantageous Effects of Invention

The present invention can provide a diaryliodonium salt having a 2-SF₅Py group.

### Brief Description of Drawings

Fig. 1 illustrates an antitumor property of a diaryliodonium salt.
Fig. 2 illustrates an antitumor property of a diaryliodonium salt.

### Description of Embodiments

Hereinafter, the present invention will be described in detail. In the present invention, "X to Y" includes values at both ends, that is, X and Y.

### 1. Diaryliodonium salt

The diaryliodonium salt of the present invention is represented by General Formula (d).

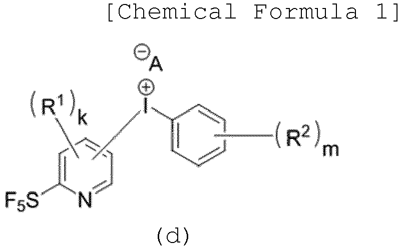

In the formula, the SF₅ group is bonded to a 2-position of the pyridine ring. The SF₅ group bonded to the 2-position lowers basicity of the pyridine ring, imparts high fat-solubility, and imparts three-dimensional bulkiness.

R¹ is a substituent on the pyridine ring and is an alkyl group having 1 or 2 carbon atoms. k represents the number of R¹ and is an integer of 0 to 4. R¹ is preferably absent, that is, k is preferably 0 from a viewpoint of reducing steric hindrance. However, in a case where R¹ is present, R¹ is preferably a methyl group, and k is preferably 1 or 2.

R² is a substituent on the benzene ring and is a linear or branched alkyl group having 1 to 4 carbon atoms. R² is preferably a methyl group, an ethyl group, an n-propyl group, an i-propyl group, or a methoxy group because of easy synthesis. m represents the number of R² and is an integer of 0 to 5. n is preferably 3 for the similar reason. In this case, R² is preferably bonded to the ortho and para positions. In a case where m is 1, R² is preferably bonded to the para position. m is preferably 0 from a viewpoint of availability of a raw material.

I is bonded to any one of 3-position to 6-position of the pyridine ring. As described later, I is preferably bonded to a 3-position or a 5-position of the pyridine ring from a viewpoint of pharmacological activity.

A⁻ is a counter anion. A⁻ affects solubility of a diaryliodonium salt in a solvent. A⁻ is preferably a trifluoromethanesulfonate anion (hereinafter, also referred to as OTf⁻) from this viewpoint.

### 2. Method for manufacturing diaryliodonium salt

A diaryliodonium salt is preferably manufactured according to the following scheme.

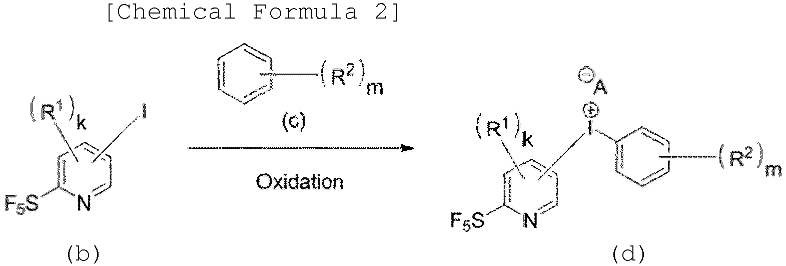

### (1) Preparation of compound (b)

First, compound (b) is prepared. R¹ and k in compound (b) are defined as described above. Compound (b) can be prepared by any method and can be prepared by an aromatic Finkelstein reaction of a corresponding Br compound in the presence of a metal catalyst (J. Am. Chem. Soc. 2002, 124, 14844., Chem. Commun. 2012, 48, 3993). Specifically, the Br-compound (compound (a)) is caused to react with NaI in the presence of CuI to obtain an I-compound. The use amount of CuI is 5 to 20 mol% relative to the Br-compound. The use amount of NaI only needs to be excessive relative to the Br-compound but is preferably 1.5 to 3 equivalents. At this time, 5 to 20 mol% of an amine relative to the Br-compound is preferably used. The amine is preferably a tertiary amine such as N,N'-dimethylethylenediamine. A solvent is not limited but is preferably an ether-based solvent such as dioxane or an alcohol-based solvent having 3 to 5 carbon atoms, such as propanol, butanol, or pentanol. A reaction temperature may be appropriately adjusted, but is preferably 80 to 150°C. The reaction is exemplified below. Even in a case where an SF₅ group and I are adjacent to each other in compound (b), synthesis is possible according to the following reaction.

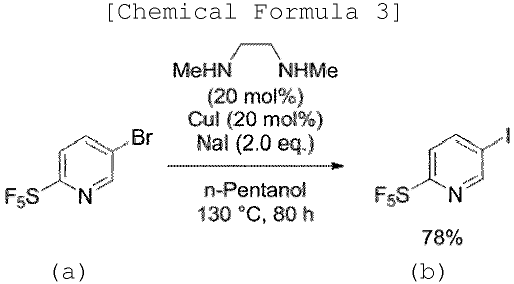

### (2) Synthesis of diaryliodonium salt (d)

An oxidation reaction of compound (b) thus prepared and a Friedel-Crafts reaction between compounds (b) and (c) are performed simultaneously. An oxidizing agent is preferably a peroxide, and particularly preferably metachloroperbenzoic acid. The use amount of the oxidizing agent is preferably 1.0 to 1.5 equivalents relative to compound (b).

Compound (c) is a benzene compound. R² and m in compound (c) are defined as above. The use amount of compound (c) is preferably 1.0 to 1.5 equivalents relative to compound (b). The Friedel-Crafts reaction is performed in the presence of a Lewis acid. The Lewis acid is preferably trifluoromethanesulfonic acid from a viewpoint of reactivity, solubility of a product in a solvent, or the like. The use amount of the Lewis acid is preferably 1.0 to 2.5 equivalents relative to compound (b).

A reaction temperature is not limited and can be about 20 to 60°C but is preferably room temperature. The solvent is preferably a halogenated hydrocarbon, and more preferably dichloromethane or the like. These reactions are described in Chem. Commun. 2007, 2521. and J. Am. Chem. Soc. 2011, 133, 13778. A diaryliodonium salt as a target compound can be purified by recrystallization using diethyl ether.

### 3. Reaction using diaryliodonium salt

A diaryliodonium salt can react with a nucleophilic compound to introduce a 2-SF₅Py group into the nucleophilic compound. This reaction can be represented by the following formula.

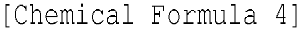

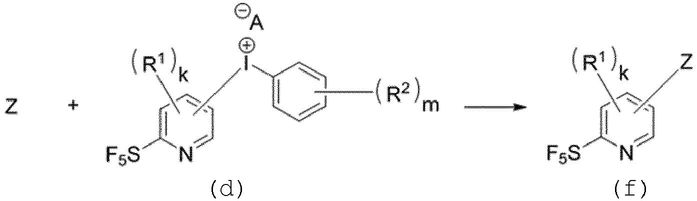

The nucleophilic compound has a nucleophilic site and can introduce an ArSF₅ group into the nucleophilic site by this reaction. Therefore, in the present invention, the nucleophilic compound is also referred to as a target compound. The reaction is preferably performed under an excessive condition such that the diaryliodonium salt is 1.1 to 2 equivalents relative to the nucleophilic compound Z. A reaction temperature can be appropriately adjusted depending on reactivity but is preferably about 20 to 160°C. A reaction solvent is also determined in consideration of the reaction temperature and solubility but is preferably a polar solvent such as water, an alcohol, NMP (N-methylpyrrolidone), DMF (N,N-dimethylformamide), or a halogenated hydrocarbon (dichloromethane or the like). A reaction accelerator such as an base or a metal can be appropriately used depending on the nucleophilic compound. Examples of the base as the reaction accelerator include a metal alkoxide such as potassium tert-butoxide and a hydrogenated metal such as sodium hydride. For the metal as the reaction accelerator, metal powder such as copper powder can be used.

Examples of the nucleophilic compound include a 1,3-dicarbonyl compound such as β-ketoester or malonic acid, a phenol compound, an aniline compound, a heterocyclic compound, an alcohol compound, an oximide compound, an aromatic sulfur compound, and an aromatic cyan compound. These compounds may each have a substituent. Examples of the phenol compound include phenol, an alkyl-substituted phenol, and a halogenated phenol. The same applies to the other compounds. In a case where a 1,3-dicarbonyl compound, a phenol compound, an alcohol compound, or an oximide compound is used, the reaction accelerator is preferably a strong base. Examples of the base include a metal alkoxide such as potassium tert-butoxide and a hydrogenated metal such as sodium hydride. In a case where an aniline compound, a heterocyclic compound, an aromatic sulfur compound, or an aromatic cyan compound is used, the reaction accelerator is preferably a metal such as copper powder.

It is estimated that a reaction mechanism of this reaction proceeds through ligand coupling as follows (ARKIVOC 2003, 43.).

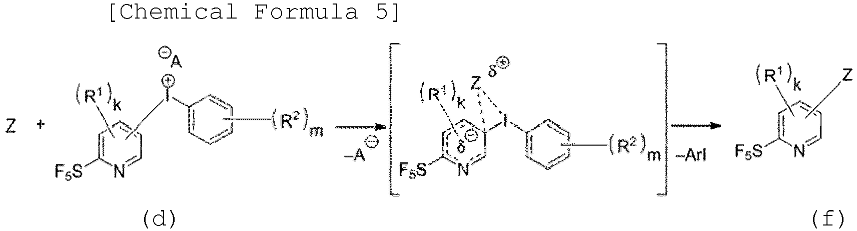

First, the nucleophilic compound Z coordinates to the central iodine I by elimination of the counter anion A. It is considered that ligand coupling with an electron-deficient 2-SF₅Py group proceeds selectively afterward through a reaction intermediate as in ipso substitution.

### 4. Pharmacological activity

The diaryliodonium salt of the present invention has an antitumor property. A tumor refers to a cell population that has started to undergo autonomous and uncontrolled proliferation by genetic mutation and includes a benign tumor and a malignant tumor. Examples of the malignant tumor include an epithelioma, a sarcoma, and a hematologic malignancy. The epithelioma is not limited, but examples thereof include skin cancer, tongue cancer, esophageal cancer, pharynx cancer, stomach cancer, colon cancer, gallbladder cancer, bile duct cancer, pancreatic cancer, duodenal cancer, kidney cancer, hepatocellular carcinoma, prostate cancer, ovarian cancer, uterine cancer, and breast cancer. The sarcoma is not limited, but examples thereof include synovial sarcoma, osteosarcoma, fibrosarcoma, liposarcoma, myosarcoma, malignant fibrous histiocytoma, dermal fibrosarcoma, angiosarcoma, Kaposi's sarcoma, and lymphangiosarcoma. The hematologic malignancy is not limited, but examples thereof include leukemia, malignant lymphoma, and multiple myeloma.

An antitumor property is evaluated by reduction or elimination of a tumor size, decrease or disappearance of the number of tumor cells, detection of apoptosis induced to tumor cells, and the like.

An antitumor agent in the present invention is a tumor therapeutic agent and can be combined with a pharmaceutical carrier to form a pharmaceutical composition. A known pharmaceutical carrier can be used, and examples thereof include a binder such as methylcellulose, a disintegrating agent such as starch, a lubricant such as magnesium stearate, a fragrance such as menthol, and a diluent such as water or physiological saline. The pharmaceutical composition can be a composition for oral administration, an injectable composition, or the like. The dosage of the pharmaceutical composition is determined in consideration of the age and weight of a subject to be administered, the type of dosage form, an administration method, and the like.

### Examples

### [Example 1]

The following reaction was performed to synthesize a diaryliodonium salt.

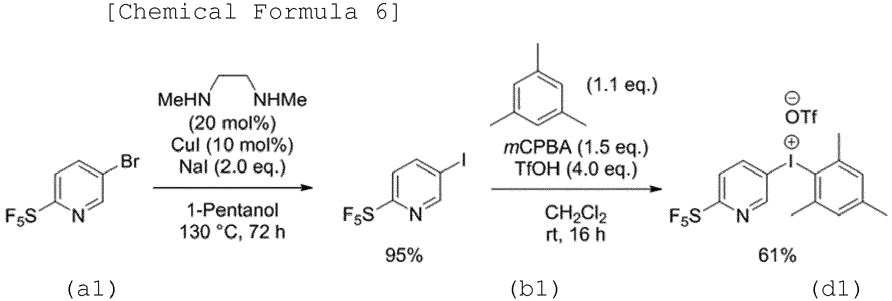

Aryl bromide (a1) (1.65 g, 5.80 mmol) synthesized by the inventors, copper (I) iodide (111 mg, 0.580 mmol, manufactured by Kanto Kagaku), and sodium iodide (1.72 g, 11.6 mmol, manufactured by Nacalai Tesque, Inc.) were put in a Schlenk tube, and the interior thereof was replaced with argon. Thereafter, N,N'-dimethylethylenediamine (0.12 mL, 1.16 mmol, manufactured by Sigma-Aldrich) and 1-pentanol (6.0 mL) were added thereto and stirred at 130°C for 72 hours. After completion of the reaction, the mixture was cooled to room temperature, and an ammonia aqueous solution (28%) and water were added thereto. The resulting mixture was extracted with dichloromethane. The organic phase was washed with a saturated sodium chloride aqueous solution and dried over sodium sulfate. Subsequently, the solvent was distilled off under reduced pressure, and the residue was purified by silica gel column chromatography (hexane/ethyl acetate = 9/1) to obtain aryl iodide (b1) (1.82 g, 95%) as a pale yellow solid.

Metachloroperbenzoic acid (77%, 1.80 g, 8.1 mmol, manufactured by Sigma-Aldrich) was put in an eggplant-shaped flask and dried under vacuum at room temperature for one hour. Thereafter, a dichloromethane solution (30 mL) of aryl iodide (b1) (1.80 g, 5.40 mmol) was added thereto. Subsequently, the resulting mixture was cooled to 0°C. Thereafter, trifluoromethanesulfonic acid (2.5 mL, 21.7 mmol, manufactured by Central Glass Co., Ltd.) was dropwise added thereto, and the resulting mixture was stirred at room temperature for two hours. Then, the mixture was cooled to 0°C. Thereafter, mesitylene (0.80 mL, 5.94 mmol, manufactured by Nacalai Tesque, Inc.) was dropwise added thereto, and the resulting mixture was further stirred at room temperature for 16 hours. After completion of the reaction, the solvent was distilled off under reduced pressure. Diethyl ether was added thereto to reprecipitate a product. The solid was collected by filtration using a Kiriyama funnel and washed with diethyl ether. Finally, the solid was dried under vacuum to obtain a diaryliodonium salt (d1) (1.97 g, 61%) as a white solid.

Analysis results by mass spectrometry and NMR are summarized below. In the present invention, mass spectrometry was performed using model name LCMS-2020 manufactured by Shimadzu Corporation, and ¹H-NMR and ¹⁹F-NMR were measured using Mercury 300 manufactured by Varian.

A diaryliodonium salt (d2) was synthesized similarly.

### [Table 1]

**Table 1 Example 1**

| Aryl bromide | Diaryliodonium salt |
|---|---|
| 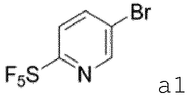 | 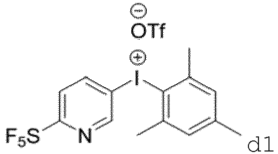 |
| | 58% Yield |
| 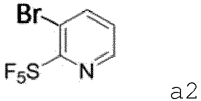 | 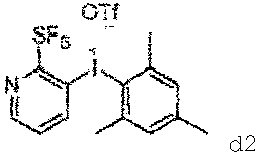 |

### Mesityl(6-(pentafluorosulfanyl)pyridin-3-yl)iodonium trifluoromethanesulfonate (d1)

MS (ESI, m/z) 450 [(M-OTf)⁺]; ¹H NMR ((CD₃)₂CO, 300 MHz): δ = 2.40 (s, 3H), 2.78 (s, 6H), 7.35 (s, 2H), 8.17 (d, J = 8.4 Hz, 1H), 8.85 (d, J = 8.4 Hz, 1H), 9.17 (s, 1H); ¹⁹F NMR (CDCl₃:DMSO-d₆ = 24:1, 282 MHz): δ = -79.0 (s, 3F), 52.1 (d, J = 149.3 Hz, 4F), 76.2 (quint, J = 149.3 Hz, 1F),; (2 steps) 58% yield

### Compound (d2)

HRMS (ESI) calcd. for C₁₄H₁₄NF₅SI [(M-OTf)⁺] : 449.9812 found 449.9812; ¹H NMR (CDCl₃, 300 MHz) δ = 2.44 (s, 3H), 2.60 (s, 6H), 7.25 (s, 2H), 7.52 (t, J = 6 Hz, 2H), 8.71 (s, 1H); ¹⁹F NMR (CDCl₃, 282 MHz) δ = -78.98 (s, 3F), 53.63 (d, J = 149.46 Hz, 4F), 75.86 (quintet, J = 152.28 Hz, 1F). (2 steps) 5% yield

### [Example 2]

The following reaction was performed to introduce a 2-SF₅Py group into a target compound.

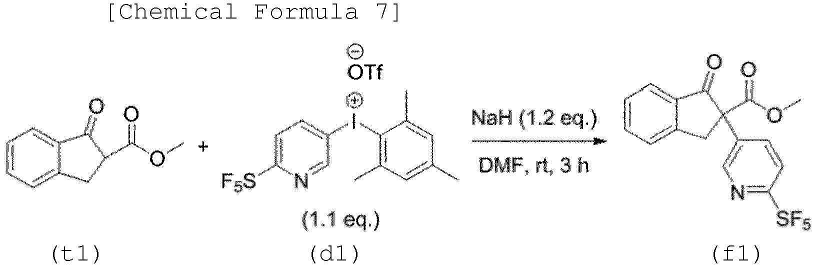

A β-ketoester (t1) synthesized by the inventors (20.7 mg, 0.109 mmol and sodium hydride (60%, 5.2 mg, 0.130 mmol, manufactured by Nacalai Tesque, Inc.) were stirred in N,N'-dimethylformamide (1.3 mL) for 10 minutes. Thereafter, the diaryliodonium salt (d1) (71.9 mg, 0.120 mmol) obtained in Example 1 was added thereto, and the resulting mixture was further stirred for three hours. After completion of the reaction, water was added thereto. The resulting mixture was extracted with diethyl ether. The organic phase was washed with a saturated sodium chloride aqueous solution and dried over sodium sulfate. Subsequently, the solvent was distilled off under reduced pressure, and the residue was purified by silica gel column chromatography (hexane/ethyl acetate = 7/3) to obtain a product (f1) (22.0 mg, 51%) as a white solid.

A similar reaction was performed using the following raw materials to manufacture products (f2) to (f6). Analysis results are summarized below.

### [Table 2]

**Table 2 Example 2**

| β-ketoester | Diaryliodonium salt | Product |
|---|---|---|
| 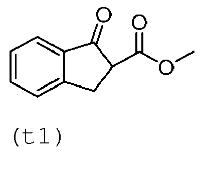 | | 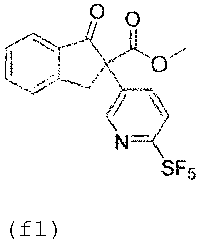 |
| 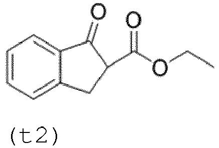 | 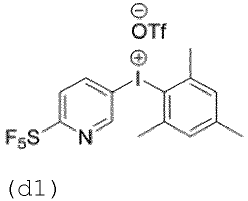 | 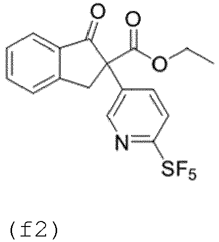 |
| 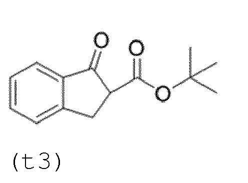 | | 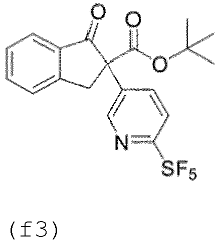 |
| 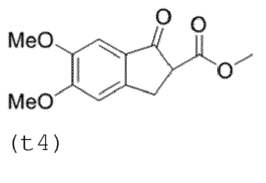 | | 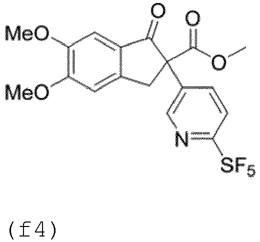 |
| 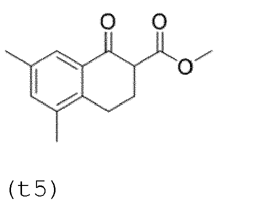 | | 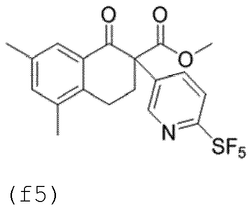 |

### Methyl 2,3-dihydro-1-oxo-2-(6-(pentafluorosulfanyl)pyridin-3-yl)-1H-indene-2-carboxylate (f1)

MS (ESI, m/z) 394 [(M+H)⁺]; ¹H NMR ((CD₃)₂CO, 300 MHz) : δ = 3.72 (s, 3H), 3.89 (d, J = 17.7 Hz, 1H), 4.26 (d, J = 17.7 Hz, 1H), 7.56 (t, J = 6.9 Hz, 1H), 7.69 (d, J = 7.2 Hz, 1H), 7.77-7.83 (m, 2H), 7.92 (d, J = 8.7 Hz, 1H), 8.31 (d, J = 7.5 Hz, 1H), 8.77 (s, 1H); ¹⁹F NMR (CDCl₃, 282 MHz) : δ = 52.7 (d, J = 148.1 Hz, 4F), 79.1 (quint, J = 148.1 Hz, 1F); 51% yield

### Ethyl 2,3-dihydro-1-oxo-2-(6-(pentafluorosulfanyl)pyridin-3-yl)-1H-indene-2-carboxylate (f2)

MS (ESI, m/z) 408 [(M+H)⁺]; ¹H NMR (CDCl₃, 300 MHz) : δ = 1.22 (t, J = 7.1 Hz, 3H), 3.63 (d, J = 17.1 Hz, 1H), 4.19-4.26 (m, 4H), 7.50 (t, J = 7.2 Hz, 1H), 7.56 (d, J = 7.5 Hz, 1H), 7.69-7.76 (m, 2H), 7.86 (d, J = 7.5 Hz, 1H), 8.17 (d, J = 8.7 Hz, 1H), 8.64 (s, 1H); ¹⁹F NMR (CDCl₃, 282 MHz) : δ = 52.1 (d, J = 149.5 Hz, 4F), 77.8 (quint, J = 149.5 Hz, 1F); 58% yield

### tert-Butyl 2,3-dihydoro-1-oxo-2-(6-(pentafluorosulfanyl)pyridin-3-yl)-1H-indene-2-carboxylate (f3)

MS (ESI, m/z) 436 [(M+H)⁺]; ¹H NMR (CDCl₃, 300 MHz) : δ = 1.38 (s, 9H), 3.61 (d, J = 17.0 Hz, 1H), 4.15 (d, J = 17.0 Hz, 1H), 7.46 (t, J = 7.4 Hz, 1H), 7.55 (d, J = 8.1 Hz, 1H), 7.67-7.75 (m, 2H), 7.84 (d, J = 8.1 Hz, 1H), 8.19 (d, J = 8.1 Hz, 1H), 8.64 (s, 1H); ¹⁹F NMR (CDCl₃, 282 MHz) : δ = 52.1 (d, J = 149.5 Hz, 4F), 77.9 (quint, J = 149.5 Hz, 1F); 72% yield

### Methyl 5,6-dimethoxy-2,3-dihydro-1-oxo-2-(6-(pentafluorosulfanyl)pyridin-3-yl)-1H-indene-2-carboxylate (f4)

MS (ESI, m/z) 454 [(M+H)⁺]; ¹H NMR (CDCl₃, 300 MHz) : δ = 3.49 (d, J = 17.0 Hz, 1H), 3.78 (s, 3H), 3.95 (s, 3H), 4.01 (s, 3H), 4.16 (d, J = 17.0 Hz, 1H), 6.95 (s, 1H), 7.25 (s, 1H), 7.75 (d, J = 8.4 Hz, 1H), 8.15 (d, J = 8.4 Hz, 1H), 8.61 (s, 1H); ¹⁹F NMR (CDCl₃, 282 MHz) : δ = 52.1 (d, J = 149.6 Hz, 4F), 77.8 (quint, J = 149.6 Hz, 1F); 67% yield Methyl 5,7-dimethyl-1-oxo-2-(6-(pentafluorosulfanyl)pyridin-3-yl)-1,2,3,4-tetrahydronaphthalene-2-carboxylate (f5)

MS (ESI, m/z) 436 [(M+H)⁺]; ¹H NMR (CDCl₃, 300 MHz) : δ = 2.34 (s, 3H), 2.35 (s, 3H), 2.67-2.79 (m, 2H), 2.89-3.09 (m, 2H), 3.24 (s, 1H), 7.80-7.84 (m, 2H), 8.50 (s, 1H); ¹⁹F NMR (CDCl₃, 282 MHz): δ = 52.1 (d, J = 149.9 Hz, 4F), 77.7 (quint, J = 149.9 Hz, 1F); 47% yield

### [Example 3]

The following reaction was performed to introduce a 2-SF₅Py group into a target compound.

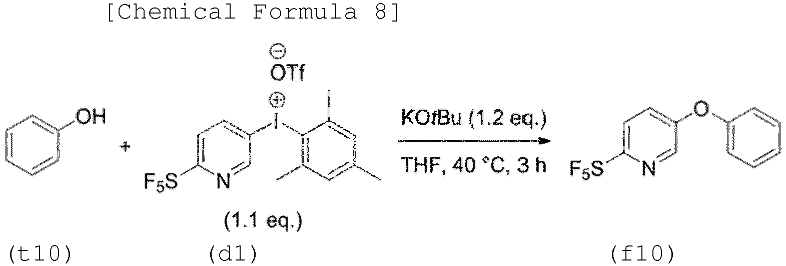

Phenol (t10) (9.4 mg, 0.100 mmol, manufactured by Tokyo Chemical Industry Co., Ltd.) was added to a tetrahydrofuran (0.3 mL) suspension of potassium tert-butoxide (13.5 mg, 0.120 mmol), and the resulting mixture was stirred at 0°C for 15 minutes. Subsequently, the diaryliodonium salt (d1) (71.9 mg, 0.120 mmol) was added to the mixture, and the resulting mixture was further stirred at 40°C for three hours. After completion of the reaction, water was added thereto, and the resulting mixture was extracted with dichloromethane. The organic phase was washed with a saturated sodium chloride aqueous solution and dried over sodium sulfate. Subsequently, the solvent was distilled off under reduced pressure, and the residue was purified by silica gel column chromatography (hexane/ethyl acetate = 9/1) to obtain a product (f10) (20.8 mg, 70%) as a white solid. Results are summarized below.

### [Table 3]

**Table 3 Example 3**

| Target compound | Diaryliodonium salt | Product |
|---|---|---|
| 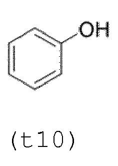 | 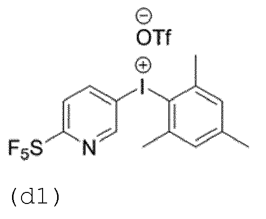 | 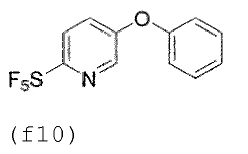 |

### 2-(Pentafluorosulfanyl)-5-phenoxypyridine (f10)

MS (EI, m/z) 297 (M⁺); ¹H NMR (CDCl₃, 300 MHz) : δ = 7.10 (d, J = 7.5 Hz, 1H), 7.26 (t, J = 7.5 Hz, 1H), 7.35-7.47 (m, 3H), 7.70 (d, J = 8.7 Hz, 1H), 8.27 (s, 1H); ¹⁹F NMR (CDCl₃, 282 MHz): δ = 53.3 (d, J = 150.0 Hz, 4F), 78.9 (quint, J = 150.0 Hz, 1F); 70% yield

### [Example 4]

The following reaction was performed to introduce a 2-SF₅Py group into a target compound.

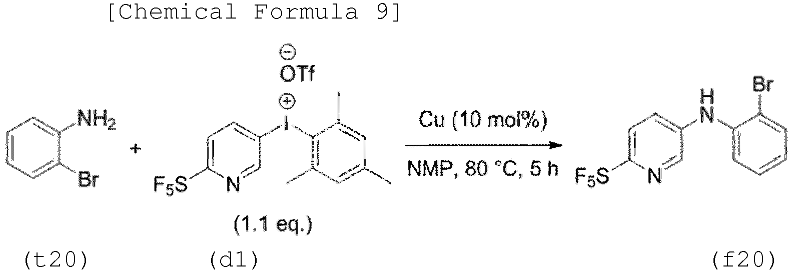

An N-methyl-2-pyrrolidone (0.4 mL) suspension of 2-bromoaniline (t20) (34.4 mg, 0.200 mmol, manufactured by Tokyo Chemical Industry Co., Ltd.) and copper powder (1.3 mg, 0.0200 mmol, manufactured by Hayashi Pure Chemical Industries) was stirred at room temperature for 10 minutes. Subsequently, the diaryliodonium salt (d1) (132 mg, 0.220 mmol) was added to the mixture, and the resulting mixture was further stirred at 80°C for five hours. After completion of the reaction, an insoluble matter was removed by silica gel filtration, and a dissolved matter was washed out with diethyl ether. Subsequently, the solvent was distilled off under reduced pressure, and the residue was purified by silica gel column chromatography (hexane/ethyl acetate = 9/1) to obtain a product (f20) (42.8 mg, 71%) as a yellow solid. Results are summarized below.

### [Table 4]

**Table 4 Example 4**

| Target compound | Diaryliodonium salt | Product |
|---|---|---|
| 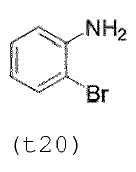 | 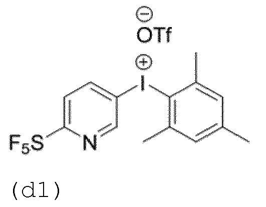 | 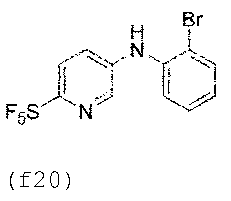 |

### N-(2-Bromopheyl)-6-(pentafluorosulfanyl)pyridine-3-amine (f20)

MS (ESI, m/z) 375 [(M+H)⁺]; ¹H NMR (CDCl₃, 300 MHz): δ = 6.24 (brs, 1H), 6.99 (t, J = 6.9 Hz, 1H), 7.30-7.46 (m, 3H), 7.62-7.65 (m, 2H), 8.27 (s, 1H); ¹⁹F NMR (CDCl₃, 282 MHz) : δ = 53.5 (d, J = 149.3 Hz, 4F), 79.9 (quint, J = 149.3 Hz, 1F); 71% yield

### [Example 5]

The following reaction was performed to introduce a 2-SF₅Py group into a target compound.

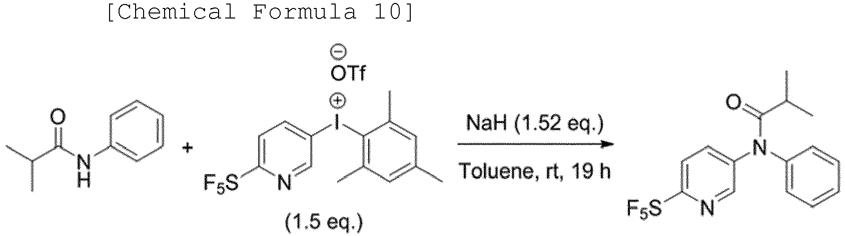

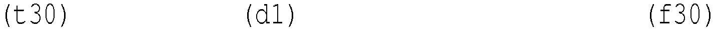

An amide (t30) (16.3 mg, 0.100 mmol) synthesized by the inventors, the diaryliodonium salt (d1) (71.9 mg, 0.120 mmol), and sodium hydride (60%, 6.1 mg, 0.152 mmol, manufactured by Nacalai Tesque, Inc.) were stirred in toluene (2.0 mL) for 19 hours. After completion of the reaction, the solvent was distilled off under reduced pressure, and the residue was purified by silica gel column chromatography (hexane/ethyl acetate = 8/2) to obtain a product (f1) (23.1 mg, 63%) as a colorless oil.

A similar reaction was performed using the following raw materials to manufacture a product (f31). Analysis results are summarized below.

### [Table 5]

**Table 5 Example 5**

| Target compound | Diaryliodonium salt | Product |
|---|---|---|
| 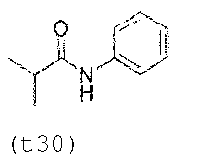 | 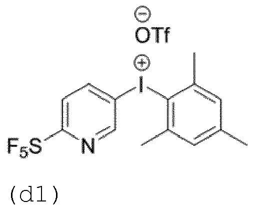 | 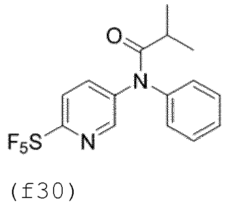 |
| 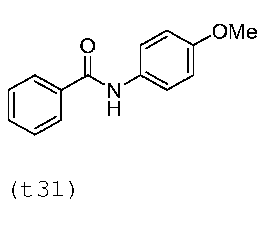 | | 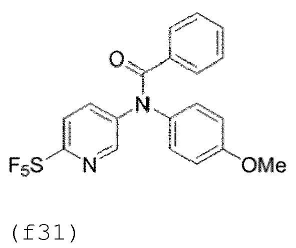 |

### N-{6-(Pentafluorosulfanyl)pyridin-3-yl}-N-phenyl isobutylamide (f30)

MS (ESI, m/z) 367 [(M+H)⁺]; ¹H NMR (CDCl₃, 300 MHz) : δ = 1.14 (d, J = 6.8 Hz, 6H), 2.67 (sept, J = 6.8 Hz, 1H), 7.27-7.29 (m, 2H), 7.46-7.54 (m, 3H), 7.69 (d, J = 8.4 Hz), 7.91 (d, J = 8.4 Hz), 8.32 (s, 1H); ¹⁹F NMR (CDCl₃, 282 MHz): δ = 52.6 (d, J = 149.3 Hz, 4F), 78.3 (quint, J = 149.3 Hz, 1F); 63% yield

### N-(4-Methoxyphenyl)-N-{6-pentafluorosulfanyl)pyridin-3-yl}benzamide (f31)

MS (ESI, m/z) 431 [(M+H)⁺]; ¹H NMR (CDCl₃, 300 MHz): δ = 3.78 (s, 3H), 6.82 (d, J = 6.8 Hz, 2H), 7.02 (d, J = 6.8 Hz, 2H), 7.24-7.29 (m, 2H), 7.25 (t, J = 7.5 Hz, 1H), 7.45 (d, J = 7.5 Hz, 2H), 7.69-7.76 (m, 2H), 8.38 (d, J = 2.4 Hz, 1H); ¹⁹F NMR (CDCl₃, 282 MHz) : δ = 52.7 (d, J = 149.7 Hz, 4F), 78.2 (quint, J = 149.7 Hz, 1F); 63% yield

According to the present invention, it is clear that a 2-pentafluorosulfanylpyridyl group can be introduced into various target compounds. In the present invention, a pentafluorosulfanylpyridyl group can be introduced into an sp3 carbon of a target compound, and the present invention is useful for a physiological substance such as this compound drug. In addition, in the present invention, a pentafluorosulfanylpyridyl group can be introduced into an oxygen atom or a nitrogen atom of a target compound.

### [Example 5]

### Pharmacological activity

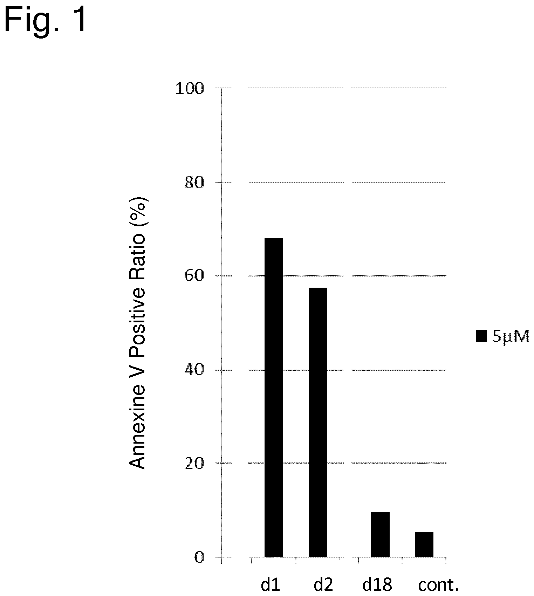

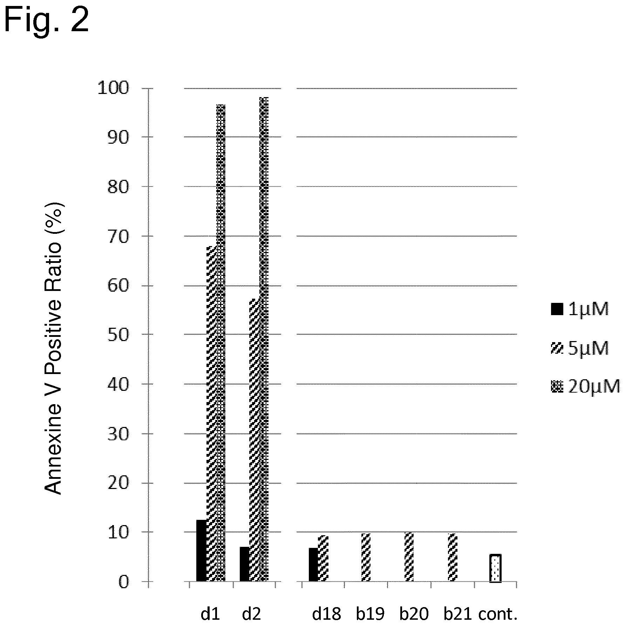

1 × 10⁴ leukemia cancer cell lines (U937: human monocytic leukemia cell line, manufactured by DS Pharma Biomedical) were suspended in 1 mL of a culture medium (RPMI 1640 medium (manufactured by Sigma-Aldrich)). The diaryliodonium salt of the present invention was dissolved in DMSO to prepare solutions of 500 µM, 2500 µM, and 10 mM. Subsequently, 2 µL of each of the solutions was dropwise added to the cancer cell line, and the solution was allowed to stand at 37°C for 24 hours. Thereafter, Annexine V positivity as an indicator of apoptosis was measured using a Muse (trademark) Annexin V and Dead Cell Assay Kit (Merck Millipore Corporation, Darmstadt, Germany) measurement kit. Results are illustrated in Figs. 1 and 2.

### [Comparative Example 1]

### Pharmacological activity

The following compounds were prepared as comparative compounds. Compound (d18) is a known substance and was synthesized by the inventors. Compound (b19) was synthesized according to the reaction described in Example 1. Compounds (b20) and (b21) were obtained from Ube Industries, Ltd. Antitumor activity was evaluated in a similar manner to Example 5 using these compounds.

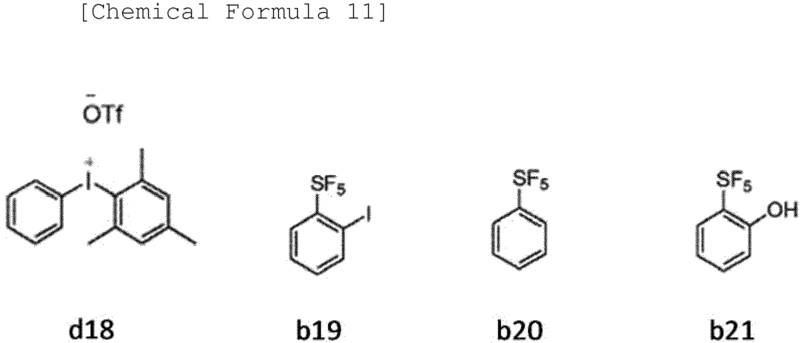

Results are illustrated in Figs. 1 and 2. It is clear that the diaryliodonium salt of the present invention exhibits an excellent antitumor property.

## Claims

1. A diaryliodonium salt having a pentafluorosulfanyl group at a 2-position of a pyridine ring, represented by General Formula (d): 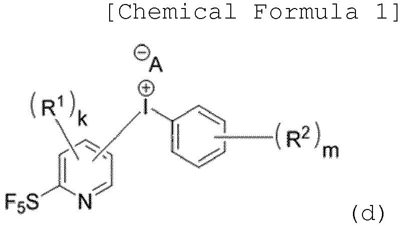 wherein
R¹ is an alkyl group having 1 or 2 carbon atoms, k is an integer of 0 to 4,
R² is a linear or branched alkyl group having 1 to 4 carbon atoms or a linear or branched alkoxy group having 1 to 4 carbon atoms, m is an integer of 0 to 5, and
A⁻ is a counter anion.

2. The diaryliodonium salt according to claim 1, wherein k is 0, and m is 3.

3. The diaryliodonium salt according to claim 2, wherein R² is a methyl group, an ethyl group, an n-propyl group, an i-propyl group, or a methoxy group.

4. The diaryliodonium salt according to claim 1, wherein k is 0, and m is 0.

5. A method for manufacturing the diaryliodonium salt according to any one of claims 1 to 4, comprising:
preparing a compound represented by General Formula (b); and
simultaneously performing an oxidation reaction of the compound and a Friedel-Crafts reaction thereof with a compound represented by General Formula (c) to generate a diaryliodonium salt represented by General Formula (d):

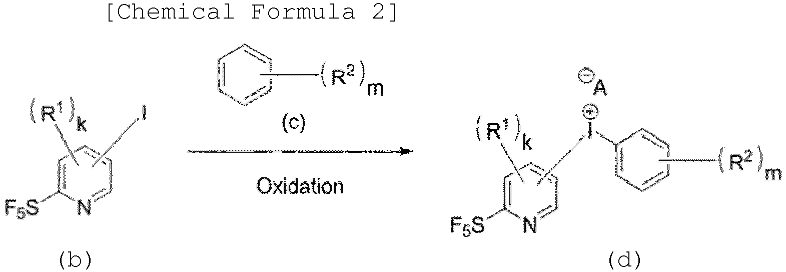

6. A method for manufacturing a compound represented by General Formula (f): 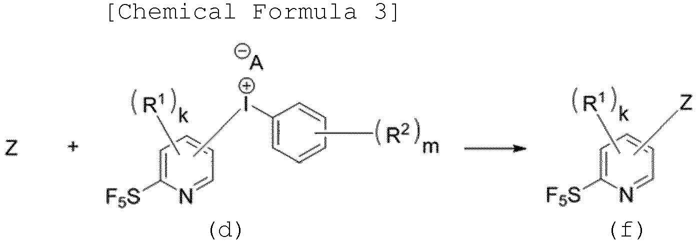 wherein R¹, k, and m are defined as described above, the method comprising
causing a reaction between a compound represented by General Formula (d) and a nucleophilic compound Z to introduce a pyridyl group having a pentafluorosulfanyl group at a 2-position into the nucleophilic compound.

7. The method for manufacturing the compound according to claim 6, wherein the nucleophilic compound Z is selected from the group consisting of a 1,3-dicarbonyl compound, a phenol compound, an aniline compound, a heterocyclic compound, an alcohol compound, an oximide compound, an aromatic sulfur compound, and an aromatic cyan compound.
